# EUROPÄISCHE PATENTANMELDUNG

(11) **EP 3 741 378 A1**
(43) Veröffentlichungstag der Anmeldung: **25.11.2020**
(21) Anmeldenummer: 19176178.2
(22) Anmeldetag: 23.05.2019
(51) Int. Cl.: A61K 36/48, A61K 36/47, A61K 36/63, A61K 8/92, A61K 9/00, A61K 31/575, A61K 36/54, A61K 36/738, A61K 47/44, A61Q 19/00, A61Q 19/10

(54) **ZUSAMMENSETZUNG ZUR TOPISCHEN BEHANDLUNG UND PFLEGE DER PSORIATISCHEN HAUT**

(71) Anmelder: Tsakouridis, Dimitrios, 08350 Barcelona (ES)
(72) Erfinder: Tsakouridis, Dimitrios, 08350 Barcelona (ES)
(74) Vertreter: Hoffmann Eitle

(57) **Zusammenfassung**

Die Erfindung betrifft eine Zusammensetzung zur topischen Behandlung und Pflege der psoriatischen Haut. Sie besteht ausschließlich aus Naturstoffen und umfasst allenfalls Erdnussöl, Olivenöl, Rizinusöl, Avocadoöl, Rosenöl, Rosenhydrolat, Lanolin sowie Phytosterol in Form einer Mischung aus wenigstens Erdnussöl, Olivenöl, Rizinusöl und Lanolin.

## Beschreibung

Psoriasis ist eine chronisch verlaufende, entzündliche, nicht-infektiöse Hautkrankheit.

Die sich bei gesunden Menschen normalerweise in einem regelmäßigen Zyklus von ca. 26-28 Tagen erneuernde Oberhaut stößt während der Erneuerung fortlaufend gealterte und dabei verhornte Hautzellen ab. Diese Erneuerung ist bei Psoriasispatienten gestört, so dass es zu einer stark beschleunigten Erneuerungsreaktion der Oberhaut kommt, die sich in scharf begrenzten, geröteten, erhobenen Hautbereichen (sogenannten Plaques) äußert, die von einer schuppenden, teilweise silbrig glänzenden Schuppenschicht bedeckt sein können. Am häufigsten sind die Gelenke (z.B. Ellenbogen, Knie), die Kopfhaut und der Genitalienbereich betroffen, aber auch die Hautanhangsgebilde wie die Nägel von Fingern oder Zehen können psoriatische Schäden aufweisen. Psoriasis geht häufig mit Begleitkrankheiten wie Arthritis oder Herz-Kreislauferkrankungen einher.

Insgesamt hängt der Verlauf und das Auftreten von sogenannten "Schüben" bei Psoriasispatienten von vielen Faktoren ab. Studien haben gezeigt, dass teilweise genetische Dispositionen beteiligt sind, jedoch auch äußere Bedingungen einen starken Einfluss haben können, so beispielsweise Infektionen, Stress, Ernährung, Wetterverhältnisse oder zusätzlich eingenommene Medikamente. Die pathologische Situation von Psoriasispatienten ist vielschichtig und geht mit einer besonders starken psychischen Belastung einher. Da die Haut als ein äußerlich sichtbares Organ des Körpers von anderen Menschen wahrgenommen wird und somit ein wichtiges Kommunikationsmittel darstellt, führt eine sichtbare Hauterkrankung, wie die Psoriasis, häufig zu einer ungerechtfertigten Zurückweisung und Ausgrenzung. Dies belastet die meisten Psoriasispatienten psychisch. Eine konsequente Behandlung der äußerlich sichtbaren Symptome ist daher grundsätzlich geboten. Neben einer systemischen Behandlung des Patienten, sind verschiedene Licht- oder Elektrotherapiemöglichkeiten bekannt. Vor allem ist jedoch die topische Behandlung von psoriatischer Haut von Bedeutung.

Bekannte Zusammensetzungen zur topischen Behandlung und Pflege der psoriatischen Haut enthalten beispielsweise Kortison, antiseptisch wirksames Dithranol (Anthralin) (so in DE 32 39 203), die Zellproliferation hemmende Vitamin D Derivate, wie Calcipotriol oder Calcitriol (so in WO2008/065514, EP2515865 oder WO2012/127037) oder Salizylsäure und Harnstoff als Keratolytika. Diese Mittel rufen jedoch bei vielen Patienten Unverträglichkeiten hervor und/oder sind aufgrund ihre Wirkungsweise ausschließlich nach ärztlicher Beratung einzusetzen und auf eine lokale Anwendung der betroffenen Stellen beschränkt.

Oft lindern Produkte mit den o.g. Substanzen die Symptome nur vorübergehend. Psoriasispatienten müssen sich bei diesen Behandlungen eine genaue Dosierung einhalten und dürfen die Produkte ausschließlich auf die betroffenen Stellen aufbringen. So kann die Haut bei einer Behandlung mit Kortison-haltigen Mitteln dünn und brüchig werden, was gerade für die bereits belasteten Psoriasispatienten von großem Nachteil ist. Dithranol-haltige Produkte können zu Verfärbungen, Hautrötungen und zusätzlichem Juckreiz auf der Haut führen, so dass bei ihnen häufig ein Abwaschen nach einer bestimmten Einwirkzeit notwendig ist. Steinkohleteer-haltige Produkte erhöhen die Empfindlichkeit der Haut für UV-Strahlen und stehen unter Verdacht, Krebs zu erregen.

Psoriasispatienten stehen derartigen Wirkstoffen daher häufig skeptisch gegenüber und suchen nach Alternativen, die sich in der Dauerbehandlung einfacher einsetzen lassen und keine der oben genannten Nebenwirkungen aufweisen. Sie sind besonders aufmerksam und kritisch gegenüber den Inhaltsstoffen von Produkten zur Pflege und Behandlung psoriatischer Haut. Insbesondere Patienten aus Risikogruppen, für die eine besondere Vorsicht bei Medikationen gilt, wie beispielsweise Kinder oder Schwangere, benötigen alternative Behandlungsmöglichkeiten.

Die Fachwelt ist daher bereits seit einiger Zeit dazu übergegangen, Produkte zur Pflege und Behandlung psoriatischer Haut auf Basis von Naturstoffen herzustellen. Naturstoffe umfassen dabei alle chemischen Verbindungen, die von Organismen gebildet werden, um eine biologische Funktion zu erfüllen. Naturstoffe können aufgrund ihrer häufig hohen strukturellen Diversität nur schwer vollsynthetisch hergestellt werden, so dass sie meist als Extrakt aus pflanzlichen oder tierischen Bestandteilen gewonnen werden. Die in Naturstoffen enthaltenen Biomoleküle umfassen biologisch aktive Substanzen, die gerade in der Pharmazeutik eine wichtige Rolle spielen.

So offenbart beispielsweise EP 3 013 423 B1 eine Zusammensetzung auf Basis von Ölen und Wachsen, mit einem öligen Auszug aus Nesseln (*Oleum coctus Urticae*)*.* Das Produkt wird unter anderem auch zur Behandlung der Haut bei Psoriasis eingesetzt.

EP 2 489 358 B1 führt den ebenfalls durch Ölextraktion gewonnenen Naturstoff Indirubin aus *Indigo naturalis* zur Behandlung der Psoriasis an. Bevorzugt werden auch hier pflanzliche Öle verwendet.

Auch EP 1 267 900 B1 nennt einen Naturstoff, Betulin, der im Wesentlichen Triterpene umfasst, welcher in Form einer Emulsion verarbeitet wird, um auf die psoriatische Haut aufgetragen zu werden.

Auch wenn die vorgenannten, auf Naturstoffen basierenden Produkte eine höhere Akzeptanz bei den Patienten haben als herkömmliche Medikamente zur Behandlung der psoriatischen Haut, sind jedoch weiterhin Nachteile in der Herstellung und der Anwendung zu verzeichnen.

Die genannten Naturstoffe werden jeweils in aufwändigen Verfahren extrahiert und weiter zu bestimmten Darreichungsformen, meist als Hautöl oder Emulsion verarbeitet. Teilweise, so beispielsweise für den Wirkstoff Betulin, müssen Lösungsmittel verwendet werden. Die Herstellungsverfahren für die Produkte sind daher aufwändig und kostenintensiv.

Auch wenn es sich um Naturstoffe handelt, können unerwünschte Nebenwirkungen nicht immer ausgeschlossen werden. So sind Unverträglichkeiten oder Überreaktionen auf Nesseln bekannt. Auch wenn bereits Verfahren zur Herstellung von Indirubin-haltigen Produkten bekannt sind, die die Hautfärbung durch Indirubin reduzieren, färbt die Substanz weiterhin die Haut. Patienten, die bereits schon aufgrund der sichtbaren Hautkrankheit psychisch belastet sind, schrecken möglicherweise auch vor dieser Art von Produkten zurück.

Der vorliegenden Erfindung liegt daher die Aufgabe zugrunde, eine Zusammensetzung zur Behandlung und Pflege der psoriatisch erkrankten Haut aus Naturstoffen bereitzustellen, welche für möglichst alle betroffenen Patienten risikoarm auf der Haut angewendet werden kann, gut verträglich ist und dabei trotzdem mit geringem Kosten- und Produktionsaufwand herzustellen ist.

Die Erfindungsaufgabe wird gelöst durch eine Zusammensetzung aus Naturstoffen gemäß Anspruch 1, welche allenfalls Erdnussöl (*Arachis hypogaea*), Olivenöl (*Olea europaea*)*,* Rizinusöl (*Ricinus communis*), Avocadoöl (*Persea gratissima*), Rosenöl (*Rosa damascena*), Rosenhydrolat, Lanolin sowie Phytosterol umfasst, wobei die Zusammensetzung in Form einer Mischung aus wenigstens Erdnussöl, Olivenöl, Rizinusöl und Lanolin vorliegt.

Erfindungsgemäß umfasst eine "Zusammensetzung aus Naturstoffen" ausschließlich natürlich vorkommende pflanzliche oder tierische Stoffe, die unverarbeitet sind oder lediglich durch mechanische oder auf Wasser basierenden Extraktionsmethoden (Flotation, Extraktion mit Wasser, Dampfdestillation usw.) gewonnen werden. Die erfindungsgemäß in der Zusammensetzung enthaltene Mischung aus wenigstens Erdnussöl, Olivenöl, Rizinusöl und Lanolin, sowie wahlweise Rosenöl und/oder Rosenhydrolat und/oder Lanolin und/oder Phytosterol umfasst demnach ausschließlich auf die oben genannte Weise gewonnene Substanzen.

Die erfindungsgemäße Zusammensetzung besteht überwiegend aus pflanzlichen Ölen, die in ihrer Mischung mit Lanolin einen höchst positiven Effekt auf die psoriatische Haut zeigen. Die Verhornungen lösen sich und die entzündete, gerötete Haut beruhigt sich. Die erfindungsgemäße Mischung ist besonders verträglich, zeigt keinerlei Nebenwirkungen und kann daher bedenkenlos von allen Patientengruppen äußerlich angewendet werden. Bei regelmäßiger Anwendung verbessert sich das Krankheitsbild, die Entzündungen der Haut gehen deutlich zurück.

Auch Psoriasispatienten, die besonderen Risikogruppen angehören, so beispielsweise Kinder und Schwangere, können die erfindungsgemäß Zusammensetzung ohne Gefahr anwenden.

Die erfindungsgemäße Zusammensetzung hat den Vorteil, dass sie die Haut nicht verfärbt, wie es bei anderen in der Psoriasisbehandlung bekannten Mitteln, beispielsweise Indigo-haltige Mittel) der Fall ist.

Die Verwendung einer geringen Zahl an Inhaltsstoffen bei der erfindungsgemäßen Zusammensetzung hat den Vorteil, dass der Patient sich einen raschen Überblick über die Inhaltsstoffe verschaffen kann. Auch das Allergierisiko wird so stark begrenzt.

Letztendlich ist die Herstellung der erfindungsgemäßen Zusammensetzung mit einer einfachen Einrichtung möglich; die Herstellungskosten sind gering.

Bevorzugt enthält die erfindungsgemäße Zusammensetzung Erdnussöl, Olivenöl, Rizinusöl und Lanolin in den Konzentrationen Erdnussöl 50-60 vol.%, Olivenöl 25-35 vol.%, Rizinusöl 8-14 vol.%, sowie Lanolin 1-4 vol.%. Die tatsächlich gewählten Konzentrationen ergeben dabei in der Summe 100 vol.% (Volumenprozent).

Ebenfalls kann die erfindungsgemäße Zusammensetzung Erdnussöl, Olivenöl, Rizinusöl, Lanolin und Rosenöl enthalten, jeweils in den folgenden Konzentrationen: Erdnussöl 50-60 vol.%, Olivenöl 25-35 vol.%, Rizinusöl 8-14 vol.%, Lanolin 1-3 vol.%, sowie Rosenöl 0,5-1,5 vol.%, wobei die gewählten Konzentrationen in der Summe 100 vol.% ergeben. Rosenöl wird mittels Wasserdampf-Destillation aus den Blütenblättern von Rosen (meist *Rosa centifolia* oder *Rosa damascena*) gewonnen. Das Rosenöl wirkt in zusammen mit den anderen Naturstoffen der erfindungsgemäßen Zusammensetzung besonders positiv auf die betroffenen Hautbereiche ein.

Gemäß einer weiteren Ausführungsform kann die erfindungsgemäße Zusammensetzung auch Erdnussöl, Olivenöl, Rizinusöl, Lanolin und Rosenhydrolat enthalten, wobei die Konzentrationen wie folgt gewählt sind: Erdnussöl 50-60 vol.%, Olivenöl 25-33 vol.%, Rizinusöl 8-14 vol.%, Lanolin 1-4 vol.%, sowie Rosenhydrolat 1-3 vol.% und in der Summe 100 vol.% ergeben. Rosenhydrolat, bzw. Rosenwasser, entsteht als Nebenprodukt bei der Destillation von Rosenöl aus Rosenblüten. Es enthält in besonders hohem Anteil 2-Phenylethanol. Rosenhydrolat wirkt auf die Haut entzündungshemmend und gleichzeitig feuchtigkeitsspendend.

Eine weitere mögliche Ausführungsform der erfindungsgemäßen Zusammensetzung enthält Erdnussöl, Olivenöl, Rizinusöl, Avocadoöl, Lanolin und Phytosterol in den folgenden Konzentrationen: Erdnussöl 50-60 vol.%, Rizinusöl 25-35 vol.%, Olivenöl 5-12 vol.%, Avocadoöl 2-8 vol.%, Lanolin 1-4 vol.%, sowie Phytosterol 0,2-0,5 vol.%, wobei die Konzentrationen der einzelnen Substanzen in der Summe 100 vol.% ergeben.

Die erfindungsgemäße Zusammensetzung kann darüber hinaus auch Erdnussöl, Olivenöl, Rizinusöl, Avocadoöl, Lanolin, Rosenöl und Phytosterol in den folgenden Konzentrationen enthalten: Erdnussöl 50-60 vol.%, Rizinusöl 25-35 vol.%, Olivenöl 3-7 vol.%, Avocadoöl 4-8 vol.%, Lanolin 2-4 vol.%, Rosenöl 0,5-1 vol.%, sowie Phytosterol 0,2-0,5 vol.%, wobei die gewählten Konzentrationen in der Summe 100 vol.% ergeben.

Erfindungsgemäß können außerdem das Rizinusöl und/oder das Olivenöl in ozonisierter Form vorliegen. Die mit Sauerstoff angereicherten Öle bewirken, dass die Haut verstärkt mit Sauerstoff versorgt wird, was die Abheilung der betroffenen Hautstellen beschleunigt.

Die erfindungsgemäße Zusammensetzung kann erfindungsgemäß auch als alleinig zur Behandlung der psoriatischen Haut wirksamer Bestandteil Inhaltsstoff eines Körperpflegemittels sein, insbesondere einer Lotion, einer Creme, einer Salbe, einer Waschlotion oder eines Schampoos.

Des Weiteren ist es im Rahmen der Erfindung vorgesehen, die Zusammensetzung auf einem Trägermaterial darzureichen, welches zur Auflage auf der zu behandelnden psoriatischen Haut vorgesehen ist und wobei das Trägermaterial eine bestimmte Menge der Zusammensetzung gemäß einem der Ansprüche 1 bis 7 zur Einwirkung auf die zu behandelnde Haut enthält. Die erfindungsgemäße Zusammensetzung kann auf diese Weise über einen längeren Zeitraum, d.h. über mehrere Stunden aus dem Arzneipflaster heraus auf die Haut einwirken, was den Behandlungserfolg weiter verbessert. Darüber hinaus wird vorteilhafterweise die Kleidung des Patienten geschont, da ein Kontakt mit der Zusammensetzung vermieden werden kann.

Im Folgenden werden fünf mögliche Ausführungsformen der Erfindung genannt:

**Tab. 1: Ausführungsform 1**

| **Ausführungsform I** | |
|---|---|
| **Inhaltsstoffe** | **Konzentration vol.%** |
| Erdnussöl (*Arachis Hypogaea*) | 55,000 |
| Rizinusöl (*Ricinus Communis*) | 30,000 |
| Olivenöl (*Olea Europaea*) | 6,500 |
| Avocadoöl (*Persea Gratissima*) | 6,000 |
| Lanolin | 2,250 |
| Phytosterol (pflanzlich) | 0,250 |

**Tab. 2: Ausführungsform 2**

| **Ausführungsform II** | |
|---|---|
| **Inhaltsstoffe** | **Konzentration vol.%** |
| Erdnussöl (*Arachis Hypogaea*) | 55,000 |
| Rizinusöl (*Ricinus Communis*) | 30,000 |
| Olivenöl (*Olea Europaea*) | 5,000 |
| Avocadoöl (*Persea Gratissima*) | 5,750 |
| Lanolin | 3,000 |
| Rosenöl (*Rosa Damascena*) | 1,000 |
| Phytosterol (pflanzlich) | 0,250 |

**Tab. 3: Ausführungsform 3**

| **Ausführungsform III** | |
|---|---|
| **Inhaltsstoffe** | **Konzentration vol.%** |
| Erdnussöl (*Arachis Hypogaea*) | 55,000 |
| ozonisiertes Rizinusöl (*Ricinus Communis*) | 30,000 |
| oznisiertes Olivenöl (*Olea Europaea*) | 10,000 |
| Lanolin | 2,400 |
| Avocadoöl (*Persea Gratissima*) | 2,350 |
| Phytosterol (pflanzlich) | 0,250 |

**Tab. 4: Ausführungsform 4**

| **Ausführungsform IV** | |
|---|---|
| **Inhaltsstoffe** | **Konzentration vol.%** |
| Erdnussöl (*Arachis Hypogaea*) | 55,000 |
| Olivenöl (*Olea Europaea*) | 30,000 |
| Rizinusöl (*Ricinus Communis*) | 12,000 |
| Lanolin | 2,000 |
| Rosenöl (*Rosa Damascena*) | 1,000 |

**Tab. 5: Ausführungsform 5**

| **Ausführungsform V** | |
|---|---|
| **Inhaltsstoffe** | **Konzentration vol.%** |
| Erdnussöl (*Arachis Hypogaea*) | 55,000 |
| Olivenöl (*Olea Europaea*) | 28,000 |
| Rizinusöl (*Ricinus Communis*) | 10,500 |
| Lanolin | 3,000 |
| Rosenwasser | 1,500 |

Die oben aufgeführten Zusammensetzungen können vorteilhafterweise mit geringem Aufwand angefertigt werden. Die einzelnen Öle werden nacheinander zusammengemischt und bei 56-58°C erwärmt. Sodann werden Lanolin und gegebenenfalls Phytosterol zugegeben und gelöst.

Die Wirkung der erfindungsgemäßen Zusammensetzungen in den oben genannten Ausführungsformen wurde bereits bei mehreren Patienten erfolgreich getestet. Die Probanden waren alle von Psoriasis Typ I betroffen. Innerhalb von 8 bis 10 Wochen konsequenter Behandlung mit der erfindungsgemäßen Zusammensetzung waren bei allen Patienten die Plaques deutlich zurückgegangen, teilweise auch vollständig abgeheilt.

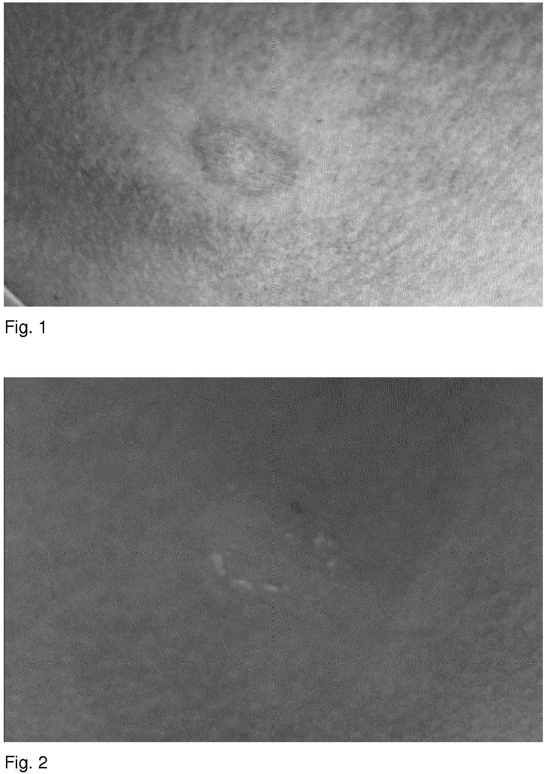

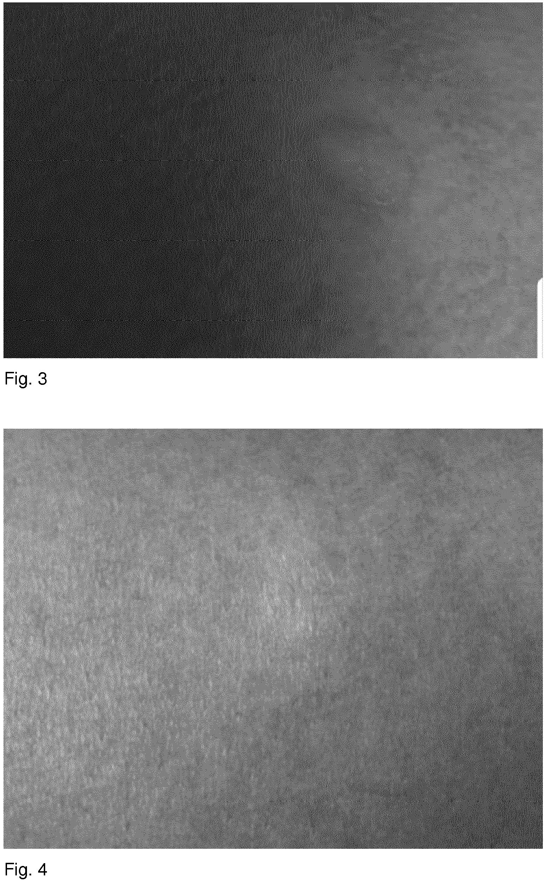

Untenstehend wird anhand eines Beispiels die Wirkung der erfindungsgemäßen Zusammensetzung erläutert. Die Patientin, 19 Jahre, ist von Psoriasis Typ I an Arm und Oberschenkel betroffen, sowie von Psoriasis capitis an der Kopfhaut. Die Plaques an Oberschenkel und Arm wurden mit der erfindungsgemäßen Zusammensetzung (Ausführungsform 4) behandelt. Die Testbehandlung begann am 15. Januar 2018 und endete am 29. März 2018. Die Zusammensetzung wurde morgens und abends auf die betroffenen Stellen aufgebracht. Die Stellen wurden mit einem Pflaster abgedeckt, und die Einwirkung der Zusammensetzung auf die Haut auch unter der Kleidung zu ermöglichen. Die Figuren 1 - 4 zeigen den Verlauf der Heilung der betroffenen Hautstelle am Oberschenkel.

Dabei zeigt
- Figur 1: die behandelte Hautstelle am Tag 1 der Behandlung,
- Figur 2: die behandelte Hautstelle am Tag 30 der Behandlung,
- Figur 3: die behandelte Hautstelle am Tag 62 der Behandlung und
- Figur 4: die behandelte Hautstelle am Tag 72 der Behandlung.

## Patentansprüche

1. Zusammensetzung zur topischen Behandlung und Pflege der psoriatischen Haut aus Naturstoffen, umfassend allenfalls Erdnussöl, Olivenöl, Rizinusöl, Avocadoöl, Rosenöl, Rosenhydrolat, Lanolin sowie Phytosterol in Form einer Mischung aus wenigstens Erdnussöl, Olivenöl, Rizinusöl und Lanolin.

2. Zusammensetzung gemäß Anspruch 1, **dadurch gekennzeichnet, dass** sie Erdnussöl, Olivenöl, Rizinusöl und Lanolin in den folgenden Konzentrationen enthält:
- Erdnussöl 50-60 vol.%,
- Olivenöl 25-35 vol.%,
- Rizinusöl 8-14 vol.%, sowie
- Lanolin 1-4 vol.%,
wobei die gewählten Konzentrationen in der Summe 100 vol.% ergeben.

3. Zusammensetzung gemäß Anspruch 1, **dadurch gekennzeichnet, dass** sie Erdnussöl, Olivenöl, Rizinusöl, Lanolin und Rosenöl in den folgenden Konzentrationen enthält:
- Erdnussöl 50-60 vol.%,
- Olivenöl 25-35 vol.%,
- Rizinusöl 8-14 vol.%,
- Lanolin 1-3 vol.%, sowie
- Rosenöl 0,5-1,5 vol.%,
wobei die gewählten Konzentrationen in der Summe 100 vol.% ergeben.

4. Zusammensetzung gemäß Anspruch 1, **dadurch gekennzeichnet, dass** sie Erdnussöl, Olivenöl, Rizinusöl, Lanolin und Rosenhydrolat in den folgenden Konzentrationen enthält:
- Erdnussöl 50-60 vol.%,
- Olivenöl 25-33 vol.%,
- Rizinusöl 8-14 vol.%,
- Lanolin 1-4 vol.%, sowie
- Rosenhydrolat 1-3 vol.%,
wobei die gewählten Konzentrationen in der Summe 100 vol.% ergeben.

5. Zusammensetzung gemäß Anspruch 1, **dadurch gekennzeichnet, dass** sie Erdnussöl, Olivenöl, Rizinusöl, Avocadoöl, Lanolin und Phytosterol in den folgenden Konzentrationen enthält:
- Erdnussöl 50-60 vol.%,
- Rizinusöl 25-35 vol.%,
- Olivenöl 5-12 vol.%,
- Avocadoöl 2-8 vol.%,
- Lanolin 1-4 vol.%, sowie
- Phytosterol 0,2-0,5 vol.%,
wobei die gewählten Konzentrationen in der Summe 100 vol.% ergeben.

6. Zusammensetzung gemäß Anspruch 1, **dadurch gekennzeichnet, dass** sie Erdnussöl, Olivenöl, Rizinusöl, Avocadoöl, Lanolin und Phytosterol in den folgenden Konzentrationen enthält:
- Erdnussöl 50-60 vol.%,
- Rizinusöl 25-35 vol.%,
- Olivenöl 3-7 vol.%,
- Avocadoöl 4-8 vol.%,
- Lanolin 2-4 vol.%,
- Rosenöl 0,5-1 vol.%, sowie
- Phytosterol 0,2-0,5 vol.%,
wobei die gewählten Konzentrationen in der Summe 100 vol.% ergeben.

7. Zusammensetzung gemäß einem der vorangehenden Ansprüche, **dadurch gekennzeichnet, dass** das Rizinusöl und/oder das Olivenöl in ozonisierter Form vorliegen.

8. Zusammensetzung gemäß einem der vorangehenden Ansprüche als alleinig zur Behandlung der psoriatischen Haut wirksamer Bestandteil eines Körperpflegemittels, insbesondere einer Lotion, einer Creme, einer Salbe, einer Waschlotion oder eines Schampoos.

9. Arzneipflaster, umfassend ein Trägermaterial zur Auflage auf der zu behandelnden psoriatischen Haut, wobei das Trägermaterial eine bestimmte Menge der Zusammensetzung gemäß einem der Ansprüche 1 bis 7 zur Einwirkung auf die zu behandelnde Haut enthält.
